# EUROPEAN PATENT APPLICATION

(11) **EP 4 726 666 A1**
(43) Date of publication of application: **15.04.2026**
(21) Application number: 24206293.3
(22) Date of filing: 12.10.2024
(51) Int. Cl.: G06V 10/25, A61B 1/00, G06T 7/00

(54) **APPARATUS AND METHOD**

(71) Applicant: Augere Medical AS, 0186 Oslo (NO)
(72) Inventor: Hagenes, Ole-Christian Schmidt, 0186 Oslo (NO); Espeland, Håvard, 0186 Oslo (NO); Petlund, Andreas, 0186 Oslo (NO); Smedsrud, Pia, 0186 Oslo (NO); de Lange, Thomas, 0186 Oslo (NO); Berstad, Tor Jan, 0186 Oslo (NO)
(74) Representative: Acapo Onsagers AS

(57) **Abstract**

A method implemented by a computer, comprising a processor and a memory, the method comprising: receiving a sequence of frames of a video; detecting a first object in the sequence of frames of the video; and signaling a first signal, responsive to detecting the first object in the sequence of frames of the video; displaying the sequence of frames of the video, comprising displaying the detected first object; and indicating a first indicator, responsive to detecting the first object in the sequence of frames of the video; wherein signaling the first signal comprises signaling the first signal concurrently with displaying the detected first object; and wherein indicating the first indicator comprises indicating the first indicator consecutively to displaying the detected first object.

## Description

### FIELD

The present invention relates to an apparatus for and a method of detecting objects, structures and/or patterns in video.

### BACKGROUND

The gastrointestinal (GI) tract (also known as GI tract, digestive tract, alimentary canal) is the tract or passageway of the digestive system from the mouth to the anus. The GI tract includes all the major organs of the digestive system, in humans and other animals, including the esophagus, stomach, and intestines. Food taken in through the mouth is digested in the GI tract to extract nutrients and absorb energy, and the waste expelled at the anus.

The GI tract is divided into upper and lower tracts, which are divided in turn. Anatomical landmarks are often used as a demarcation between the divisions. For example, the pylorus connects the stomach to the duodenum while the cardia forms the connection of the esophagus to the stomach.

The GI tract comprises four layers: the mucosa (epithelium, lamina propria, and muscular mucosae), the submucosa, the muscularis propria (inner circular muscle layer, intermuscular space, and outer longitudinal muscle layer) and the serosa. The mucosa is the innermost layer and is in direct contact with the luminal contents of the GI tract, such as food (undigested, partially digested and/or digested) and stool. The appearance and/or change of appearance of the mucosa may be symptomatic of a disease. For example, abnormalities such as atypical texture and/or color, erosions, ulcerations, vascular malformations, inflammation, hemorrhages or polyps or and/or on the mucosa may be symptomatic of a disease such as Barrett's esophagus, stomach ulcers and/or inflammatory bowel diseases (IBD), such as Crohn's disease or ulcerative colitis or colon cancer.

Using an endoscope such as a colonoscope, a gastroscope or a duodenoscope, the appearance of the mucosa may be visually examined by a physician. During an endoscopy, the physician views, on a display, a live video that is being concurrently acquired by the endoscope camera (i.e. live video in real time). The appearance of the mucosa may be visually examined by a physician during the insertion phase and/or the withdrawal phase of an endoscopy. However, patient discomfort may be relatively greater during the insertion phase and/or a conformation of the GI tract may change between the insertion phase and withdrawal phase. Hence, the appearance of the mucosa is typically visually examined by a physician during the withdrawal phase of an endoscopy. Responsive to viewing a suspected abnormality in the live video, the physician may perform an action in real time, for example a recordal action (such as store an image of the suspected abnormality and/or log the suspected abnormality) and/or a remedial action (such as obtain a biopsy of the suspected abnormality, treat the suspected abnormality and/or excise the suspected abnormality).

Some abnormalities may be readily visually apparent to a physician during an endoscopy. However, some abnormalities may not be readily apparent such that these abnormalities are missed (i.e. false negatives) during an endoscopy. For example, an abnormality may not be readily apparent due to the conformation of the GI tract and/or structure, such as folds and curves, of the GI tract. For example, an abnormality may not be readily apparent due to its appearance, morphology, size and/or location. For example, an abnormality may not be readily apparent due to obscuring, for example partially obscuring or fully obscuring, such as by food or stool, for example resulting from deficient preparation for the endoscopy. For example, an abnormality may not be readily apparent due to transient imaging thereof by the endoscope camera such that the abnormality is viewable for only a relatively short duration for example at most 1 s or at most 0.5 s or less. For example, an abnormality may not be readily apparent due to a speed and/or orientation of the endoscope camera. For example, an abnormality may not be readily apparent due to a direction of movement (insertion or withdrawal) of the endoscope camera. For example, an abnormality may not be readily apparent due to illumination, reflection and/or shadowing during the endoscopy. For example, an abnormality may not be readily apparent due to a skill level and/or error of the physician.

Consequently, 19% to 26% of polyps may be missed (i.e. false negatives) during colonoscopy, for example. As of 2012, colorectal cancer is the second most common cause of cancer in women and the third most common in men. In 2018, 1.8 million people were diagnosed with colorectal cancer and 881,000 people died from it. As more than 80% of colorectal cancers arise from adenomatous polyps, detection and removal of such polyps is crucial for preventing them to turn into cancer.

Computer-implemented methods have been proposed to address some of the limitations due to a skill level and/or error of the physician. These computer-implemented methods seek to improve the accuracy (i.e. to reduce the rate of false negatives and/or false positives) of detecting abnormalities, for example polyps. For example, a known computer-implemented method provides real-time detection of polyps during a colonoscopy, in which a detected polyp is signaled using a bounding box overlaid on the video.

However, there remains a need to improve methods of endoscopy. More generally, there remains a need to improve, for example, medical imaging, video surveillance and/or video inspection.

### SUMMARY OF THE INVENTION

A first aspect provides a method implemented by a computer, comprising a processor and a memory, the method comprising:
receiving a sequence of frames of a video;
detecting a first object in the sequence of frames of the video;
signaling a first signal, responsive to detecting the first object in the sequence of frames of the video;
displaying the sequence of frames of the video, comprising displaying the detected first object; and
indicating a first indicator, responsive to detecting the first object in the sequence of frames of the video;
wherein signaling the first signal comprises signaling the first signal concurrently with displaying the detected first object; and
wherein indicating the first indicator comprises indicating the first indicator consecutively to displaying the detected first object.

A second aspect provides a computer, comprising a processor and a memory, configured to perform the method according to the first aspect.

A third aspect provides a computer program comprising instructions which, when executed by a computer, comprising a processor and a memory, cause the computer to perform the method according to the first aspect.

A fourth aspect provides a tangible computer-readable recording medium storing instructions which, when executed by a computer, comprising a processor and a memory, cause the computer to perform the method according to the first aspect.

A fifth aspect provides an apparatus comprising a camera, a display and a computer, comprising a processor and a memory, configured to perform the method according to the first aspect.

### DETAILED DESCRIPTION OF THE INVENTION

### Method

The first aspect provides a method implemented by a computer, comprising a processor and a memory, the method comprising:
receiving a sequence of frames of a video;
detecting a first object in the sequence of frames of the video;
signaling a first signal, responsive to detecting the first object in the sequence of frames of the video;
displaying, for example on a display, the sequence of frames of the video, comprising displaying the detected first object; and
indicating a first indicator, responsive to detecting the first object in the sequence of frames of the video;
wherein signaling the first signal comprises signaling the first signal concurrently with displaying the detected first object; and
wherein indicating the first indicator comprises indicating the first indicator consecutively to displaying the detected first object.

In this way, the first object is detected by the method implemented by the computer, thereby improving an accuracy (i.e. reducing a rate of false negatives and/or false positives) of detecting objects in the sequence of frames of the video.

In this way, the detected first object is signaled, for example to a user, concurrently with displaying thereof. For example, the detected first object may be signaled, for example to a user, while the detected first object is being displayed and/or during display of the detected first object.

In this way, the detected first object is indicated, for example to a user, consecutively to displaying thereof. For example, the detected first object may be indicated, for example to a user, after the detected first object was displayed and/or when the detected first object is no longer displayed. In this way, the user is continuously guided to the detected first object since the detected first object is indicated by the first indicator consecutively to displaying thereof, for example after the detected first object was displayed and/or when the detected first object is no longer displayed. In this way, a likelihood of the user missing the detected first object is reduced. For example, in this way, a likelihood of a physician missing a detected polyp during a colonoscopy is reduced.

### Method

In one example, the method comprises and/or is a method of processing video, for example live video. In one example,

In one example, the method comprises and/or is a method of video surveillance. In one example, the method comprises and/or is a method of video inspection (also known as internal inspection).

In one example, the method comprises and/or is a method of medical imaging, for example endoscopy, colonoscopy, bronchoscopy, gastroscopy, cystoscopy, ERCP, laparoscopy, arthroscopy, laryngoscopy, sigmoidoscopy, hysteroscopy, capsule endoscopy, endoscopic ultrasound, enteroscopy, esophogealgastroduodenoscopy, colposcopy, percutaneous endoscopy, thoracoscopy, ureteroscopy, ablation, chromoendoscopy, endoscopic mucosal resection and neuroendoscopy. In one example, the method comprises and/or is a method of colonoscopy.

In one example, the method comprises and/or is a method of processing medical imaging video, for example video acquired from endoscopy, colonoscopy, bronchoscopy, gastroscopy, cystoscopy, ERCP, laparoscopy, arthroscopy, laryngoscopy, sigmoidoscopy, hysteroscopy, capsule endoscopy, endoscopic ultrasound, enteroscopy, esophogealgastroduodenoscopy, colposcopy, percutaneous endoscopy, thoracoscopy, ureteroscopy, ablation, chromoendoscopy, endoscopic mucosal resection and neuroendoscopy. In one example, the method comprises and/or is a method of processing endoscopy video for example colonoscopy video.

### Computer

The method is implemented by the computer, comprising the processor and the memory. Suitable computers are known.

In one example, the computer comprises and/or is a local computer for an apparatus comprising a camera and a display. In this way, the computer may be provided for the apparatus.

In one example, the computer comprises and/or is an external computer, for example an external local computer, bidirectionally or unidirectionally communicatively coupled to an apparatus comprising a camera and a display. In this way, the computer may be included with an existing apparatus.

In one example, the computer comprises and/or is an internal computer, for example an internal local computer, included with an apparatus comprising a camera and a display. In this way, the computer may be included with a new apparatus.

In one example, the computer comprises and/or is a remote computer, for example a server, bidirectionally or unidirectionally communicatively coupled to an apparatus comprising a camera and a display.

### Sequence of frames

The method comprises receiving the sequence of frames of the video.

It should be understood that the video includes the sequence of frames. It should be understood that the sequence of frames includes a plurality of sequential frames. It should be understood that a frame comprises and/or is a static image. In one example, a frame of the sequence of frames comprises an image region, for example corresponding with a view imaged by a camera, and a non-image region, for example a border surrounding, at least in part, the image region. For example, endoscopic video typically provides a circular image region and a surrounding square or rectangular non-image region.

It should be understood that the frames in the sequence of frames are in correct sequential order (i.e. correct temporal order) and are received in the correct sequential order. In one example, receiving the sequence of frames of the video comprises receiving the sequence of frames of the video at a frame rate in a range from 0.001 FPS to 1,000 FPS, preferably in a range from 10 FPS to 100 FPS, more preferably in a range from 24 FPS to 60 FPS. Typically, a frame rate of at least 24 FPS is perceived as fluid motion by a human viewer. Increasing the frame rate above 60 FPS increases computer resource requirements while fluidity of motion to a human viewer is not typically further improved.

In one example, a frame of the sequence of frames has a resolution in a range from 640 x 360 to 7680 x 2160, such as nHD, VGA, SVGA, XGA, WXGA, SXGA, HD, WXGA+, HD+, UXGA, WSXGA+, FHD, WUXGA, WQXGA, QXGA, UWFHD, QHD, WQXGA, UWQHD, 4K UHD or DUHD.

In one example, the video comprises and/or live video. Live video is non-prerecorded, ongoing video imaging of events currently in process. Examples of live video include video acquired during video surveillance, video inspection and/or medical imaging. In this way, the sequence of frames of the video are live (i.e. current). In one example, the video comprises and/or live streaming video, for example streamed over the internet. In one example, the video comprises and/or live broadcast video, for example transmitted by a broadcast station. In one example, the video is being concurrently acquired by a camera such as an endoscope camera (i.e. live video in real time). In one example, the method comprises recording the sequence of frames of the live video, such as completely or partially (i.e. non-transitory live video). In one example, the method comprises not recording (i.e. excludes recording) the sequence of frames of the live video (i.e. transitory live video). If the sequence of frames of the live video is recorded, the recorded video may be reviewed. Conversely, if the sequence of frames of the live video is not recorded, the recorded video may not be reviewed, such that the method may not be repeated on the sequence of frames of the video. That is, the method is preferably performed in real time, particularly for transitory live video. In one example, the method comprises capturing a frame, for example a selected frame, of the sequence of frames of the video, for example non-transitory live video or transitory live video.

In one example, receiving the sequence of frames of the video comprises transitorily storing the sequence of frames of the video, such as receiving a sequence of frames of live video. In this way, the sequence of frames of the video are transitorily stored, for example temporarily stored such as in a buffer or in RAM, rather than non-transitorily stored, for example permanently stored such as on a HDD. In one example, receiving the sequence of frames of the video excludes non-transitorily storing the sequence of frames of the video.

In one example, the video comprises and/or is endoscopic video, for example gastrointestinal tract endoscopic video such as colonoscopy video, and wherein the first detected object comprises and/or is an endoscopic feature for example erythema, erosion, ulceration, edema, nodules, polyp and/or stenosis and/or an anatomical structure selected from a group consisting of healthy mucosa, stool, colonic fluid, blood vessel, inflamed mucosa, erosion, lesion and polyp.

In one example, the method comprises acquiring the sequence of frames of the video, for example using a camera. In one example, the method comprises transmitting the acquired sequence of frames of the video to the computer, for example via wireless transmission and/or wired transmission.

In one example, acquiring the sequence of frames of the video comprises acquiring the sequence of the frames during an insertion phase and/or during a withdrawal phase of video inspection and/or medical imaging, for example endoscopy.

In one example, receiving the sequence of frames of the video comprises intercepting the sequence of frames of the video. For example, the computer may be configured to intercept the acquired sequence of frames between a camera, configured to acquire the sequence of frames of the video, and a display, configured to display the acquired sequence of frames of the video.

### Objects

The method comprises detecting the first object in the sequence of frames of the video. More generally, the method comprises detecting an object, a structure and/or a pattern in the sequence of frames of the video.

In one example, the first object comprises and/or is an anatomical structure, for example a part or a structure inside a human or an animal body, which may be visually examined via endoscopy. For example, anatomical structures of the GI tract include the interior surface of the GI tract, including healthy mucosa, stool, fluids like gastric fluid or colonic fluid and blood vessels but also abnormal and/or diseased tissue or structures such as abnormal mucosa, e.g. inflamed mucosa, erosions, lesions (e.g. ulcers) or polyps (i.e. abnormalities). The anatomical structures may be contrast-enhanced anatomical structures, for example provided using a contrast agent. For example, mucosa may be dyed with a dye such as methylene blue, that may be sprayed onto the mucosa to assist identifying dysplasia or pre-cancerous lesions.

In one example, the first object comprises and/or is an anatomical landmark, for example a region, a part or a structure inside a human or an animal body, which may be visually examined via endoscopy and which forms and/or provides a demarcation, such as between two adjacent regions, part or structures. For example, anatomical landmarks of the GI tract include the pylorus (which connects the stomach to the duodenum), the cardia (which forms the connection of the esophagus to the stomach), the cecum (which marks the beginning of the large intestine) and the z-line (which marks the beginning of the beginning of the gastric mucosa and can be seen as an irregular zig-zag line).

### Detecting objects

The method comprises detecting the first object in the sequence of frames of the video.

It should be understood that detecting the first object is implemented by the computer. That is, the computer detects the first object in the sequence of frames of the video i.e. automated detection. In one example, detecting the first object in the sequence of frames of the video comprises machine vision and/or computer vision. In one example, detecting the first object in the sequence of frames of the video comprises object detection. Methods of object detection are known, including neural network approaches and non-neural approaches.

In one example, detecting the first object in the sequence of frames of the video is as described in WO 2020/256568 A1, included herein in entirety by reference.

In one example, detecting the first object in the sequence of frames of the video comprises: applying a sliding window to the sequence of frames and for each position of the sliding window, extracting one or more visual features from said frames within the sliding window, thereby generating a plurality of time images;
applying a trained classifier to each time image, wherein the trained classifier determines one or more detection scores that estimate the likelihoods that the respective time image contains the first object; and
outputting in real-time the detection of the first object when the detection score is higher than a detection threshold of the trained classifier.

In one example, the size of the sliding window is fixed or dynamic. In one example, the sliding window is overlapping or non-overlapping. In one example, the sliding rate of the sliding window and frame rate of the input video are identical. In one example, the one or more visual features are extracted by employing algorithms for local feature extraction and/or global feature extraction and/or deep feature extraction. In one example, the visual features are deep features and such deep features are extracted through deep neural networks (DNN). In one example, supervised methods are used for the DNN to extract such deep features. In one example, unsupervised methods are used for the DNN to extract such deep features. In one example, the classifier is trained for multi-class classification. In one example, the classifier is a DNN or a capsule network adapted for analyzing images. The resolution of a time image depends on the amount of data in said time image, i.e. a large amount of data results in a high resolution while a small amount of data results in a low resolution. The resolution of the time image affects processing time. For example, a time image of at least 1024 x 768 pixels (i.e. XGA) may be processed in real-time. In one example, the size of the sliding window is fixed, e.g. the sliding window includes 2, 3, 4, 5, 6 or more frames. The size of the sliding window affects the resolution of the time image generated for each position of the sliding window. For a given number of visual features, a smaller size sliding window, e.g. a sliding window that includes 2 or 3 frames, results in time images having a lower resolution than if the applied sliding window had a larger size, e.g. 5 or 6. In one example, the size of the sliding window is dynamic, i.e. its size varies with time. This may be useful to achieve a trade-off between resolution and processing time, e.g. when the region of the GI tract that is currently under examination is not of particular interest for polyp detection, but segmentation information is required by the user. The method may apply a sliding window that results in a lower resolution of the time image, which is sufficient for segmentation, while for polyp detection in a segment of interest, the system may apply a sliding windowthat results in a higher resolution of the time image required for a reliable polyp detection. The sliding window be an overlapping or a non-overlapping sliding window. A non-overlapping sliding window will include different frames for each of its positions, e.g. a non-overlapping sliding window including 5 frames will at its first position include frames 1 to 5, at its second position frames 6 to 10, at its third position frames 11 to 15 and so on. Another example of a non-overlapping sliding window is one that skips one or more frames. For example, a sliding window may be applied which includes 5 frames and which skips each 6th frame, i.e. the first position of the sliding window is frame 1 to frame 5, the second position of the sliding window is frame 7 to 11, the third position of the sliding window is frame 13 to 17 and so on. An overlapping sliding window may overlap by one or more frames. For example, a sliding window may be applied which includes 5 frames and which overlaps by 4 frames, i.e. the first position of the sliding window is frame 1 to frame 5, the second position frame 2 to frame 6, the third position frame 3 to frame 7 and so on. For example, a sliding window may be applied which includes 5 frames and which overlaps by 1 frame, i.e. the first position of the sliding window covers frame 1 to frame 5, the second position frame 4 to frame 8, the third position frame 7 to frame 11 and so on. In one example, the sliding rate of the sliding window and frame rate of the input endoscopic video are identical. For each position of the sliding window, one or more visual features from said frames within the sliding window are extracted and a time image, i.e. the two-dimensional representation of the visual features extracted from the sequence of frames covered by the sliding window at each position, is generated. In one example, more than one visual feature is extracted. Visual features are those features by which the trained classifier identifies the first object. For feature extraction, different families of algorithms, namely local features, global features and deep features, may be employed. For local features, suitable feature descriptors include Scale-Invariant Feature Transform (SIFT), Maximally Stable Extremal Regions (MSER), Features From Accelerated Segment Test (FAST), Speeded Up Robust Features (SURF), Center Surround Extremas (CENSURE), Binary Robust Invariant Scalable Key-points (BRISK) and Fast Retina Key-point (FREAK). For global features, suitable feature descriptors include Color and Edge Directivity Descriptor (CEDD), Joint Composite Descriptor (JCD), Auto Color-Correlogram, Color-Fayout, Edge-Histogram, Rotation Invariant Local Binary Patterns, (Pyramid of) Histograms of Orientation Gradients and Tamura. The advantages with using local and global features are that they are easy and fast to calculate. In one example, deep features are extracted through deep neural networks (DNN), e.g. convolutional deep neural networks. DNNs are machine learning based neural networks with multiple hidden layers of learned features or variables between the input layer and the output layer. In one example, supervised methods are used for the DNN to extract the visual features. In one example, unsupervised methods are used for the DNN to extract the visual features, such as variational autoencoders or generative adversarial networks. In one example, for visual feature extraction, local features and global features are employed. In one example, local features and deep features are employed. In one example, global and deep features are employed. In one example, local features, global features and deep features are employed. In one example, a feature buffer is added that caches computed features from previous positions of the sliding window for reuse when the window slides to a new position/set of frames. The addition of such a feature buffer avoids recomputing features multiple times, improves throughput and contributes to decrease computation time. In one embodiment, the trained classifier is a DNN adapted for analyzing images or a capsule network adapted for analyzing images and trained to identify said anatomical structures and/or landmarks. The DNN may be trained using known methods. In one example, the DNN is trained using a dataset of time images having ground truth labels associated with the one or more anatomical structures and/or one or more anatomical landmarks. Any suitable approach can be used to divide the time image dataset. For example, a randomly selected half of the time image dataset can be used for training the DNN and the remaining half of the time image dataset can be used to test the DNN. In another preferred embodiment, the decision layer of the network is used to compute its performance and learn a threshold of detection score for classification using cross-validation. The resolution of the time image may be changed prior to being fed into the classifier. By way of example, time images may initially be fed into the classifier with a lower resolution (than their acquired resolution) and provided to the classifier at a higher resolution if the classifier identifies an anatomical structure and/or anatomical landmark of interest. Alternatively, the classifier may process time images of a lower resolution (e.g. disregard features and/or frames) and process time images of a higher resolution when a change of feature values suggests that there is anatomical structure and/or anatomical landmark of interest. In one example, the steps of applying the sliding window and applying the trained classifier are carried out in parallel in real time. In one example, a frame rate of the sequence of frames received is at least 30 FPS and the steps of applying the sliding window and applying the trained classifier are carried out in parallel in real-time. Each detection score is compared to a detection threshold learned by the trained classifier during said training. In response to the determination that a detection score for a class in the time image is higher than the learned detection threshold, the classification of the time image by the trained classifier is output in real time. In one example, the first signal is signaled (for example, output) in real-time to the user. The first signal may be in the form of a visual alert, e.g. displayed on a display. For example, a label can be used to denote the presence and/or the location of the anatomical structure and/or landmark, either by overlaying the video or separately from the video, e.g. above, below or beside. The label may be in the form of a color code, a text, a sign or a shape. Additionally and/or alternatively, the first signal may be in the form of an audio alert and/or a haptic alert. In one example, a visual alert, an audio alert and/or a haptic alert are combined. For example, an audio alert may denote the presence of a polyp in the colon while the presence of stool may be denoted by a visual alert such as a color coding in the endoscopic video. The output may also include the detection score(s) for the detected one or more anatomical structures and/or landmarks. In one example, the output may further include instructions or recommendations to the user for adapting the procedure, such as repeating imaging of or zooming in on a particular anatomical location, or further examining a previous anatomical location. In one example, the classification results are stored on a memory or storage of a computer system. For example, if the endoscopic video is from a capsule endoscopy where the capsule's motion cannot be controlled. In one example, the first signal is signaled in real-time to the user and the classification results are stored on a memory or storage of a computer system. This allows the user to review and assess the stored information, for example after an endoscopic procedure has been finalized and to e.g. copy in or transfer images with identified anatomical structures such as polyps into the patient's record. An example of computer software is overlay software that enables the method to overlay the output over the video acquired by the endoscope, such that an overlaid video is shown on a display to the user carrying out the endoscopic procedure. Thus, the overlaid video may comprise an annotated sequence of frames in the form of a visual detection signal indicative of e.g. a polyp. Another example of such computer software is fail-safe software, i.e. software ensuring that the video feed acquired by the endoscope - and the information on the position of the endoscope monitored by the positioning system, if present - are displayed on a display in real-time to the user carrying out the endoscopic procedure even if the method fails. In one example, such a fail-safe may be implemented as a hardware component present in the computing device. In one example, such hardware component is a video compositor, i.e. a memory buffer comprised in the graphics hardware. The video compositor preferably operates independently from the processor, ensuring that the video feed acquired by the endoscope - and the information on the position of the endoscope monitored by the positioning system, if present - is displayed on a display in real-time to the user carrying out the endoscopic procedure even if the method fails. In one example, the video compositor is implemented on a field-programmable gate array. While the method allows for real-time detection of one or more anatomical structures and/or one or more anatomical landmarks while the endoscopic video is still being received, it will nevertheless be appreciated that while the detection is real-time, it is not necessarily instantaneous and that there may be processing delays/latencies, of a few milliseconds, e.g. more than 10, 20, 30 or 40 ms or even more than 100 ms. In one example, the latency is addressed by the video compositor overlaying the results of the processing over the most recent frames of the endoscopic video feed. This leads to a few frames misalignment between the processing and the endoscopic video feed, which is usually acceptable but may lead to mismatches if there is very fast movement in the endoscopic video feed. In one example, the video compositor buffers the endoscopic video feed until the received sequence of frames has been processed before overlaying the results of said processing over the endoscopic video feed. This may lead to a latency of more than 100 ms, e.g. 150 ms or 200 ms, but ensures that the overlay matches in a situation where there is very fast movement in the endoscopic video. In one example, the video compositor is configured to either buffer or overlay the results over the most recent frames, i.e. configured to work in either of the two modes described above. In one example, method comprises selecting a mode the video compositor is working based on the type of information that is to be presented to the user.

### Displaying

The method comprises displaying, for example on a display, the sequence of frames of the video, comprising displaying the detected first object.

In one example, the method comprises: compositing the sequence of frames of the video and the first signal and/or the first indicator, thereby providing a composite video; and wherein displaying the sequence of frames of the video, comprising displaying the detected first object, comprises displaying the composited video. In this way, a composite video may be output by the computer for display on a display.

### Signaling

The method comprises signaling, for example outputting, the first signal, responsive to detecting the first object in the sequence of frames of the video. It should be understood that the first signal is signaled in response to detecting the first object. That is, detecting the first object causes signaling of the first signal whereby the first signal corresponds with detection of the first object.

Signaling the first signal comprises signaling the first signal concurrently (i.e. simultaneously) with displaying the detected first object. That is, the detected first object is displayed and the first signal is signaled at the same time, for example for the same time duration (i.e. same start time and end time). In one example, signaling the first signal concurrently with displaying the detected first object comprises signaling the first signal only concurrently with displaying the detected first object. That is, the first signal is signaled only while the detected first object is displayed and hence for the same time duration.

In one example, signaling the first signal comprises transiently signaling the first signal. For example, the first signal may be signaled for the time equivalent of a relatively small number of frames, for example in a range from 1 to 120 frames such as at a frame rate of 24 FPS to 60 FPS. For example, the first signal may be signaled for a time in a range from 0.02 s to 2 s, preferably in a range from 0.1 s to 1 s, more preferably in a range from 0.2 s to 0.5 s, for example 0.2 s or 0.3 s or 0.4 s or 0.5 s. For example, the first object may be detected transiently whereby the first signal is signaled transiently.

In one example, the first signal comprises and/or is and/or is provided in the form of a visual signal (for example a visual alert), an audio signal (for example an audio alert) and/or a haptic signal (for example a haptic alert), as described previously. In this way, the first signal may be signaled to a user, for example a human user.

In one example, the visual signal comprises and/or is a colour code, a text, a sign and/or a shape. In one example, the shape comprises and/or is a bounding box or parts thereof such as corners and/or sides, adapted (for example sized) to surround, at least in part, the detected first object, for example without occlusion thereof. In one example, a colour of the shape represents a class of the detected first object.

In one example, the first signal comprises and/or is and/or is provided in the form of a control signal. In this way, the first signal may be signaled to a user, for example a human user and/or a non-human user such as to control performing an action in real time. In one example, the action comprises and/or is a null action (such as cancel the first signal), a control action (such as to control the method for example control acquiring of the video), a recordal action (such as capture a frame of the sequence of frames including the first object and/or log the first object, such as a presence, an identity and/or a location thereof) and/or a remedial action (such as related to detecting the first object e.g. initiate washing, obtain a biopsy of a suspected abnormality, treat a suspected abnormality and/or excise a suspected abnormality).

In one example, signaling the first signal concurrently with displaying the detected first object comprises displaying the first signal concurrently with displaying the detected first object, for example wherein the first signal comprises and/or is and/or is provided in the form of a visual signal. In this way, the detected object and the first signal are displayed concurrently, for example on the same display.

In one example, the method comprises compositing the sequence of frames of the video and the first signal, for example by overlaying the first signal and the sequence of frames of the video such as overlaying the first signal on the sequence of frames of the video, thereby providing a composite video. In this way, the composite video may be output by the computer for display on a display.

In one example, displaying the first signal concurrently with displaying the detected first object comprises overlaying the first signal and the sequence of frames of the video. In this way, the first signal may be provided by overlaying the sequence of frames of the video, for example.

In one example, overlaying the first signal and the sequence of frames of the video comprises not occluding the detected first object. In this way, the detected first object is not occluded by the first signal whereby the detected first object may be viewed without occlusion thereof.

In one example, a frame of the sequence of frames comprises an image region, including the detected first object, and a non-image region, wherein overlaying the first signal and the sequence of frames of the video comprises overlaying the image region. In this way, the detected first object is signaled in the image region.

### Indicating

The method comprises indicating the first indicator, responsive to detecting the first object in the sequence of frames of the video. It should be understood that the first indicator is indicated in response to detecting the first object. That is, detecting the first object causes indicating of the first indicator whereby the first indicator corresponds with detection of the first object.

Indicating the first indicator comprises indicating the first indicator consecutively (i.e. subsequently) to displaying the detected first object. That is, the detected first object is displayed first and the first indicator is indicated at a consecutive time thereafter, for example for a subsequent time duration (i.e. the indicating start time is the same as the displaying end time). In one example, indicating the first indicator consecutively to displaying the detected first object comprises indicating the first indicator only consecutively to displaying the detected first object.

That is, the first indicator is indicated only after, for example immediately after, the detected first object is displayed. In one example, indicating the first indicator consecutively to displaying the detected first object comprises indicating the first indicator concurrently with and consecutively to displaying the detected first object. That is, the first indicator is indicated while and after the detected first object is displayed.

In one example, indicating the first indicator comprises persistently indicating the first indicator. For example, the first indicator may be indicated for the time equivalent of a relatively large number of frames, for example in a range from 24 to 600 frames such as at a frame rate of 24 FPS to 60 FPS. For example, the first indicator may be indicated for a time in a range from 0.5 s to 10 s, preferably in a range from 1 s to 5 s, more preferably in a range from 2 s to 4 s, for example 2 s or 3 s or 4 s. For example, the first object may be detected transiently while the first indicator is indicated persistently. In this way, a likelihood of a user missing the detected first object is further reduced.

In one example, signaling the first signal comprises transiently signaling the first signal and indicating the first indicator comprises persistently indicating the first indicator. For example, the first object may be detected transiently whereby the first signal is signaled transiently while the first indicator is indicated persistently. In this way, a likelihood of a user missing the detected first object is further reduced.

In one example, signaling the first signal and indicating the first indicator comprises consecutively signaling the first signal and indicating the first indicator. In this way, the first indicator is indicated after the first signal is signaled. In one example, signaling the first signal and indicating the first indicator comprises concurrently signaling the first signal and indicating the first indicator and consecutively signaling the first signal and indicating the first indicator. In this way, the first indicator is indicated while the first signal is signaled and after the first signal is signaled.

In one example, the first indicator comprises and/or is and/or is provided in the form of a visual indicator (for example a visual alert), an audio indicator (for example an audio alert) and/or a haptic indicator (for example a haptic alert), as described with respect to the first signal mutatis mutandis. In this way, the first indicator may be indicated to a user, for example a human user.

In one example, the visual indicator comprises and/or is a colour code, a text, a sign and/or a shape. In one example, the shape comprises and/or is a flag, arranged (for example positioned) to point to the detected first object, for example without occlusion thereof and/or without occlusion of an image region. In one example, a colour of the shape represents a class of the detected first object and/or a brightness of the shape represents a separation between the current frame in the sequence of frames and the preceding frame, including the detected first object.

In one example, the first indicator comprises and/or is and/or is provided in the form of a control indicator. In this way, the first indicator may be indicated to a user, for example a human user and/or a non-human user such as to control performing an action in real time. In one example, the action comprises and/or is a null action (such as cancel the first indicator), control action (such as to control the method for example control acquiring of the video), a recordal action (such as capture a frame of the sequence of frames including the first object and/or log the first object, such as a presence, an identity and/or a location thereof) and/or a remedial action (such as related to detecting the first object e.g. initiate washing, obtain a biopsy of a suspected abnormality, treat a suspected abnormality and/or excise a suspected abnormality).

In one example, indicating the first indicator comprises indicating the first indicator until an action is initiated, performed and/or completed. In this way, indicating the first indicator persists until the action is initiated, performed and/or completed. In this way, a likelihood of a user failing to initiate an action for the detected first object is reduced.

In one example, the first signal and the first indicator are mutually different, for example mutually distinguishable and/or provided in mutually different forms. In this way, a user may differentiate between newly detected objects and previously detected objects.

In one example, indicating the first indicator comprises pointing from the current frame (i.e. the currently displayed frame) in the sequence of frames towards a preceding frame in the sequence of frames. In this way, a user is further continuously guided to the detected first object. In one example, indicating the first indicator comprises pointing from the current frame in the sequence of frames towards a preceding frame, including the detected first object, in the sequence of frames.

In one example, indicating the first indicator comprises indicating the first indicator based on a separation, for example a temporal separation and/or a spatial separation, between the current frame in the sequence of frames and a preceding frame, including the detected first object, in the sequence of frames. In this way, a user is further continuously guided to the detected first object since indicating the first indicator is based on the separation. For example, an attribute of the first indicator may be relatively larger at a relatively smaller separation while the attribute of the first indicator may be relatively smaller at a relatively larger separation, or vice versa. For example, the further in time and/or distance the current frame is from the frame including the detected first object, the smaller the attribute of the first indicator, or vice versa. In this way, a likelihood of the user missing the detected first object is reduced. In one example, an attribute of the first indicator comprises and/or is: a size, a shape, a colour, a pattern, a luminance and/or a brightness for a visual indicator; a frequency, an amplitude, a speed, a direction, a duration, a pitch, an intensity (or loudness) and/or a timbre for an audio indicator; and/or a frequency, an amplitude and/or a pattern for a haptic indicator.

In one example, indicating the first indicator based on the separation between the current frame in the sequence of frames and the preceding frame, including the detected first object, in the sequence of frames comprises and/or is changing an attribute of the first indicator based on the separation between the current frame in the sequence of frames and the preceding frame, including the detected first object, in the sequence of frames. In this way, a user is further continuously guided to the detected first object since indicating the first indicator is changed based on the separation.

In one example, indicating the first indicator based on the separation between the current frame in the sequence of frames and the preceding frame, including the detected first object, in the sequence of frames comprises and/or is changing an attribute of the first indicator inversely proportionally to the separation between the current frame in the sequence of frames and the preceding frame, including the detected first object, in the sequence of frames. In this way, a user is further continuously guided to the detected first object since indicating the first indicator is changed inversely based on the separation.

In one example, the separation comprises and/or is a temporal separation (for example, time separation and/or number of frames separation) and/or a spatial separation (for example, distance).

In one example, detecting the first object in the sequence of frames of the video comprises: detecting the first object in a first sub-sequence of the sequence of frames of the video; and not detecting the first object in a successive second sub-sequence of the sequence of frames of the video; wherein signaling the first signal comprises signaling the first signal concurrently (optionally, only concurrently) with displaying the first sub-sequence of the sequence of frames of the video; and wherein indicating the first indicator comprises indicating the first indicator concurrently with displaying the successive second sub-sequence of the sequence of frames of the video. In this way, the first indicator indicates the first object while or even though the detected object is not displayed.

In one example, signaling the first signal concurrently with displaying the first sub-sequence of the sequence of frames of the video comprises signaling the first signal only concurrently with displaying the first sub-sequence of the sequence of frames of the video.

In one example, indicating the first indicator comprises indicating the first indicator concurrently with displaying the first sub-sequence of the sequence of frames of the video and concurrently with displaying the successive second sub-sequence of the sequence of frames of the video.

In one example, detecting the first object in the sequence of frames of the video comprises: detecting the first object in a first frame of the sequence of frames of the video; and not detecting the first object in a successive second frame of the sequence of frames of the video; wherein signaling the first signal comprises signaling the first signal concurrently (optionally, only concurrently) with displaying the first frame of the sequence of frames of the video; and wherein indicating the first indicator comprises indicating the first indicator concurrently with displaying the successive second frame of the sequence of frames of the video. In this way, the first indicator indicates the first object while or even though the detected object is not displayed.

In one example, signaling the first signal concurrently with displaying the first frame of the sequence of frames of the video comprises signaling the first signal only concurrently with displaying the first frame of the sequence of frames of the video.

In one example, indicating the first indicator comprises indicating the first indicator concurrently with displaying the first frame of the sequence of frames of the video and concurrently with displaying the successive second frame of the sequence of frames of the video.

In one example, indicating the first indicator consecutively to displaying the detected first object comprises displaying the first indicator consecutively to displaying the detected first object. In this way, the first indicator indicates the first object while or even though the detected object is not displayed.

In one example, indicating the first indicator consecutively to displaying the detected first object comprises displaying the first indicator consecutively to displaying the detected first object, for example wherein the first indicator comprises and/or is and/or is provided in the form of a visual indicator. In this way, the detected object and the first indicator are displayed consecutively, for example on the same display.

In one example, the method comprises compositing the sequence of frames of the video and the first indicator, for example by overlaying the first indicator and the sequence of frames of the video such as overlaying the first indicator on the sequence of frames of the video, thereby providing a composite video. In this way, the composite video may be output by the computer for display on a display.

In one example, displaying the first indicator consecutively to displaying the detected first object comprises overlaying the first indicator and the sequence of frames of the video. In this way, the first indicator may be provided by overlaying the sequence of frames of the video, for example.

In one example, overlaying the first indicator and the sequence of frames of the video comprises not occluding the detected first object. In this way, the detected first object is not occluded by the first indicator whereby the detected first object may be viewed without occlusion thereof.

In one example, a frame of the sequence of frames comprises an image region, including the detected first object, and a non-image region, wherein overlaying the first indicator and the sequence of frames of the video comprises overlaying the non-image region. In this way, the image region is not obscured by the first indicator whereby the image region, including the detected first object, may be viewed without obscuring thereof.

In one example, the method comprises:
detecting a second object in the sequence of frames of the video;
signaling a second signal, responsive to detecting the second object in the sequence of frames of the video; and
indicating a second indicator, responsive to detecting the second object in the sequence of frames of the video;
wherein displaying the sequence of frames of the video comprises displaying the detected second object;
wherein signaling the second signal comprises signaling the second signal concurrently with displaying the detected second object;
wherein indicating the second indicator comprises indicating the second indicator consecutively to displaying the detected second object; and
wherein indicating the first indicator comprises indicating the first indicator concurrently with displaying the detected second object.

In this way, indicating of the previously-detected first object and displaying and signaling of the newly-detected second object are concurrent. In other words, the previously-detected first object continues to be indicated, even though not displayed, while the newly-detected second object is displayed and signaled. In this way, a likelihood of a user missing the detected first object is further reduced, even is a second object is detected.

In one example, the first signal and the second signal are mutually different, for example mutually distinguishable and/or provided in mutually different forms. In this way, a user may differentiate between newly detected objects and previously detected objects.

In one example, the first indicator and the second signal are mutually different, for example mutually distinguishable and/or provided in mutually different forms. In this way, a user may differentiate between newly detected objects and previously detected objects.

### Other applications

The method is not limited to medical imaging videos such as endoscopic videos, for example, but may be used for the real-time detection of objects, structures and/or patterns in any type of video, for example video surveillance and/or video inspection. For example, the method may be used for the real-time detection of objects, structures and/or patterns in videos obtained from private or public surveillance cameras. An example is the use for facial classification, e.g. for identification of wanted criminals in smart cities or blacklisted or restricted persons at schools, hospitals or in private companies. Another example is the detection of unattended baggage on airports, train- or subway stations. Further, the method may be used for the real-time detection of objects, structures and/or patterns in videos obtained from satellites or drones. An example is the use for early detection of natural disasters, such as wildfires, floods and volcanic eruptions. Another example is the detection of sharks in proximity to beaches, e.g. by helping to detect sharks in water with low visibility and to distinguish them from other objects of similar size, shape and color, e.g. seals or even to distinguish a harmless shark (e.g. a plankton-eating shark) from a shark which can be potentially dangerous to swimmers and surfers. The method may also be used in aquaculture, e.g. fish farming, where videos surveillance of fish in fish cages or ponds is used to assess the activity level of the fish. Typically, fish that display low activity, e.g. swim slowly, do not feed and fish feed needs to be added to obtain the desired growth rates and weight. As of today, an operator watches the video and determines fish activity, but with the method of the invention, the determination of activity levels, their assessment and also subsequent actions as a result of the assessment, e.g. the addition of fish feed, can be automatized. The method may also be used for quality control purposes. Today, quality controls are often still carried out manually, e.g. by workers at an assembly line. The method may be used to detect structures or patters indicative of damaged or sub-standard quality goods on a video obtained from the goods on the assembly line.

### Computer, computer program and/or tangible computer-readable recording medium

The second aspect provides a computer, comprising a processor and a memory, configured to perform the method according to the first aspect.

The third aspect provides a computer program comprising instructions which, when executed by a computer, comprising a processor and a memory, cause the computer to perform the method according to the first aspect.

The fourth aspect provides a tangible computer-readable recording medium, for example a tangible non-transitory computer-readable recording medium, storing instructions which, when executed by a computer, comprising a processor and a memory, cause the computer to perform the method according to the first aspect.

### Apparatus

The fifth aspect provides an apparatus comprising a camera, a display and a computer, comprising a processor and a memory, configured to perform the method according to the first aspect.

In one example, the camera is configured to acquire the sequence of frames of the video and to transmit the acquired sequence of frames of the video to the computer.

In one example, the display is configured to display the sequence of frames of the video.

In one example, the camera is configured to acquire the sequence of frames of the video and to transmit the acquired sequence of frames of the video to the display. In one example, the computer is configured to: intercept the transmitted sequence of frames of the video; perform the method according to the first aspect; and transmit composited video to the display.

In one example, the apparatus comprises and/or is an endoscopy apparatus comprising an endoscope including the camera.

In one example, the endoscopy apparatus comprises an endoscope including the camera and a light source, a camera controller and the display. The endoscopy apparatus may further include a data storage unit for storing video. The endoscope may further include one or more channels for air, suction, administration of medication, contrast agents, dyes or optical imaging agents or for allowing the insertion of medical instruments for manipulating the part or region of the body under examination, e.g. for taking biopsies or the removal of polyps or lesions. In one example, the endoscope may be in the form of a swallowable device capable of acquiring a video of e.g. the GI tract as it passes through the body. In one example, the endoscope includes a positioning system configured to determine a position of the tip or end of the endoscope or a swallowable device (e.g. a capsule) configured to determine a position thereof.

### BRIEF DESCRIPTION OF THE DRAWINGS

For a better understanding of the invention, and to show how exemplary embodiments of the same may be brought into effect, reference will be made, by way of example only, to the accompanying diagrammatic Figures, in which:
Figure 1 schematically depicts a method according to an exemplary embodiment;
Figure 2 schematically depicts a method according to an exemplary embodiment;
Figure 3 schematically depicts a method according to an exemplary embodiment; and
Figure 4 schematically depicts an apparatus according to an exemplary embodiment.

### DETAILED DESCRIPTION OF THE DRAWINGS

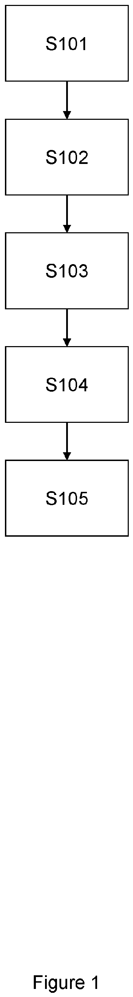

Figure 1 schematically depicts a method according to an exemplary embodiment.

The method is implemented by a computer, comprising a processor and a memory. The method comprises:
receiving a sequence of frames of a video (S101);
detecting a first object in the sequence of frames of the video (S102); and signaling a first signal, responsive to detecting the first object in the sequence of frames of the video (S103);
displaying the sequence of frames of the video, comprising displaying the detected first object (S104); and
indicating a first indicator, responsive to detecting the first object in the sequence of frames of the video (S105);
wherein signaling the first signal comprises signaling the first signal concurrently with displaying the detected first object; and
wherein indicating the first indicator comprises indicating the first indicator consecutively to displaying the detected first object.

The method may include any step described with respect to the first aspect.

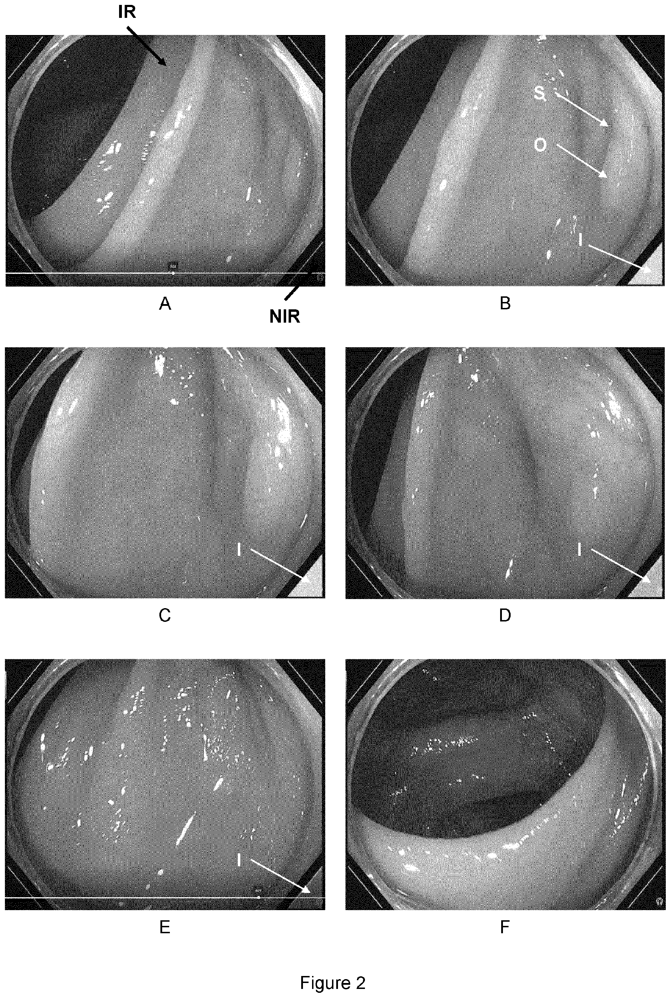

Figure 2 schematically depicts a method according to an exemplary embodiment. Particularly, Figure 2 schematically depicts a method of processing medical imaging video, specifically colonoscopy video, according to an exemplary embodiment. In more detail, Figure 2 shows a sequence of six (6) frames A to F of the colonoscopy video.

Frame A shows the mucosa of the colon in an imaging region IR of the frame A. Frame A also has a non-imaging region NIR.

Frame B includes a transiently detected first object O, particularly a polyp, disposed towards the bottom right corner of the frame B. Frame B includes a first signal S, provided by green corners of a bounding box. Frame B includes a first indicator I, provided by a solid green triangle in the bottom right corner of the frame B and thereby pointing spatially towards the first object O.

Frame C no longer includes the previously detected first object O. Frame C continues to include the first indicator I, provided by the solid green triangle in the bottom right corner of the frame C and thereby pointing spatially and temporally towards the previously detected first object O.

Frame D does not include the previously detected first object O. Frame D continues to include the first indicator I, provided by the faded green triangle in the bottom right corner of the frame D and thereby pointing spatially and temporally towards the previously detected first object O.

Frame E does not include the previously detected first object O. Frame E continues to include the first indicator I, provided by the further faded green triangle in the bottom right corner of the frame E and thereby pointing spatially and temporally towards the previously detected first object O.

Frame F does not include the previously detected first object O.

In more detail, the method is implemented by a computer, comprising a processor and a memory, the method comprising:
receiving a sequence of frames A to F of a video;
detecting a first object O in the sequence of frames of the video;
signaling a first signal S, responsive to detecting the first object O in the sequence of frames of the video;
displaying, for example on a display, the sequence of frames of the video, comprising displaying the detected first object O; and
indicating a first indicator I, responsive to detecting the first object O in the sequence of frames of the video;
wherein signaling the first signal S comprises signaling the first signal S concurrently with displaying the detected first object O; and
wherein indicating the first indicator I comprises indicating the first indicator I consecutively to displaying the detected first object O.

In this example, the method comprises and/or is a method of processing video, for example live video. In this example, the method comprises and/or is a method of colonoscopy. In this example, the method comprises and/or is a method of processing endoscopy video for example colonoscopy video.

In this example, the computer comprises and/or is a local computer for an apparatus comprising a camera and a display.

In this example, a frame of the sequence of frames comprises an image region IR, for example corresponding with a view imaged by a camera, and a non-image region NIR, for example a border surrounding, at least in part, the image region IR. In this example, the colonoscopy video provides a quasi-circular image region IR and a surrounding square non-image region NIR including four (4) triangular non-image regions at respective corners thereof.

In this example, receiving the sequence of frames of the video comprises receiving the sequence of frames of the video at a frame rate in a range from 24 FPS to 60 FPS. In this example, a frame of the sequence of frames has a resolution of 1024 x 768 pixels i.e. XGA. In this example, the video comprises and/or live video. In this example, the video is being concurrently acquired by an endoscope camera (i.e. live video in real time). In this example, the method comprises not recording (i.e. excludes recording) the sequence of frames of the live video (i.e. transitory live video). In this example, the method comprises capturing a frame, for example a selected frame, of the sequence of frames of the video, for example non-transitory live video or transitory live video.

In this example, receiving the sequence of frames of the video comprises transitorily storing the sequence of frames of the video, such as receiving a sequence of frames of live video. In this example, receiving the sequence of frames of the video excludes non-transitorily storing the sequence of frames of the video.

In this example, the method comprises acquiring the sequence of frames of the video, for example using a camera. In this example, the method comprises transmitting the acquired sequence of frames of the video to the computer, for example via wireless transmission and/or wired transmission.

In this example, acquiring the sequence of frames of the video comprises acquiring the sequence of the frames during a withdrawal phase of the colonoscopy

In this example, the video comprises colonoscopy video and the first detected object comprises and/or is an endoscopic feature for example erythema, erosion, ulceration, edema, nodules, polyp and/or stenosis and/or an anatomical structure selected from a group consisting of healthy mucosa, stool, colonic fluid, blood vessel, inflamed mucosa, erosion, lesion and polyp.

In this example, receiving the sequence of frames of the video comprises intercepting the sequence of frames of the video.

In this example, the first object O comprises and/or is an anatomical structure for example erythema, erosion, ulceration, edema, nodules, polyp and/or stenosis and/or an anatomical structure selected from a group consisting of healthy mucosa, stool, colonic fluid, blood vessel, inflamed mucosa, erosion, lesion and polyp.

In this example, detecting the first object O in the sequence of frames of the video comprises computer vision. In this example, detecting the first object O in the sequence of frames of the video comprises object detection. In this example, detecting the first object O in the sequence of frames of the video is as described in WO 2020/256568 A1, included herein in entirety by reference.

In this example, detecting the first object O in the sequence of frames of the video comprises: applying a sliding window to the sequence of frames (for example over time) and for each position (for example each time position) of the sliding window, extracting one or more visual features from said frames (for example a plurality of said frames) within the sliding window (for example to generate a respective time image), thereby generating a plurality of time images; applying a trained classifier to each time image, wherein the trained classifier determines one or more detection scores that estimate the likelihoods that the respective time image contains the first object O; and
outputting in real-time the detection of the first object O when the detection score is higher than a detection threshold of the trained classifier.

In this example, the method comprises: compositing the sequence of frames of the video and the first signal S and/or the first indicator I, thereby providing a composite video; and wherein displaying the sequence of frames of the video, comprising displaying the detected first object O, comprises displaying the composited video.

In this example, signaling the first signal S concurrently with displaying the detected first object O comprises signaling the first signal S only concurrently with displaying the detected first object O.

In this example, signaling the first signal S comprises transiently signaling the first signal S.

In this example, the first signal S comprises and/or is and/or is provided in the form of a visual signal (for example a visual alert).

In this example, the visual signal comprises and/or is a shape. In this example, the shape is a bounding box (particularly corners thereof), adapted (for example sized) to surround, at least in part, the detected first object O, for example without occlusion thereof. In this example, a colour of the shape represents a class of the detected first object O.

In this example, signaling the first signal S concurrently with displaying the detected first object O comprises displaying the first signal S concurrently with displaying the detected first object O, for example wherein the first signal S comprises and/or is and/or is provided in the form of a visual signal.

In this example, the method comprises compositing the sequence of frames of the video and the first signal S, by overlaying the first signal S and the sequence of frames of the video such as overlaying the first signal S on the sequence of frames of the video, thereby providing a composite video.

In this example, displaying the first signal S concurrently with displaying the detected first object O comprises overlaying the first signal S and the sequence of frames of the video.

In this example, overlaying the first signal S and the sequence of frames of the video comprises not occluding the detected first object O.

In this example, a frame of the sequence of frames comprises an image region IR, including the detected first object O, and a non-image region NIR, wherein overlaying the first signal S and the sequence of frames of the video comprises overlaying the image region IR. In this way, the detected first object O is signaled in the image region IR.

In this example, indicating the first indicator I consecutively to displaying the detected first object O comprises indicating the first indicator I concurrently with and consecutively to displaying the detected first object O.

In this example, indicating the first indicator I comprises persistently indicating the first indicator I. For example, the first indicator I may be indicated for the time equivalent of a relatively large number of frames, for example in a range from 24 to 600 frames such as at a frame rate of 24 FPS to 60 FPS. For example, the first object O may be detected transiently while the first indicator I is indicated persistently.

In this example, signaling the first signal S comprises transiently signaling the first signal S and indicating the first indicator I comprises persistently indicating the first indicator I.

In this example, signaling the first signal S and indicating the first indicator I comprises concurrently signaling the first signal S and indicating the first indicator I and consecutively signaling the first signal S and indicating the first indicator I.

In this example, the first indicator I comprises and/or is and/or is provided in the form of a visual indicator (for example a visual alert).

In this example, the visual indicator comprises is a shape. In this example, the shape comprises and/or is a flag, arranged (for example positioned) to point to the detected first object O, for example without occlusion thereof and/or without occlusion of an image region IR. In this example, a colour of the shape represents a class of the detected first object O and a brightness of the shape represents a separation between the current frame in the sequence of frames and the preceding frame, including the detected first object O.

In this example, the first signal S and the first indicator I are mutually different, for example mutually distinguishable and/or provided in mutually different forms.

In this example, indicating the first indicator I comprises pointing from the current frame (i.e. the currently displayed frame) in the sequence of frames towards a preceding frame in the sequence of frames. In this example, indicating the first indicator I comprises pointing from the current frame in the sequence of frames towards a preceding frame, including the detected first object O, in the sequence of frames.

In this example, indicating the first indicator I comprises indicating the first indicator I based on a separation, for example a temporal separation and/or a spatial separation, between the current frame in the sequence of frames and a preceding frame, including the detected first object O, in the sequence of frames. In this example, an attribute of the first indicator I comprises and/or is: a brightness for a visual indicator.

In this example, indicating the first indicator I based on the separation between the current frame in the sequence of frames and the preceding frame, including the detected first object O, in the sequence of frames comprises and/or is changing an attribute of the first indicator I based on the separation between the current frame in the sequence of frames and the preceding frame, including the detected first object O, in the sequence of frames.

In this example, indicating the first indicator I based on the separation between the current frame in the sequence of frames and the preceding frame, including the detected first object O, in the sequence of frames comprises and/or is changing an attribute of the first indicator I inversely proportionally to the separation between the current frame in the sequence of frames and the preceding frame, including the detected first object O, in the sequence of frames.

In this example, the separation comprises and/or is a temporal separation (for example, time separation and/or number of frames separation).

In this example, detecting the first object O in the sequence of frames of the video comprises: detecting the first object O in a first sub-sequence of the sequence of frames of the video; and not detecting the first object O in a successive second sub-sequence of the sequence of frames of the video; wherein signaling the first signal S comprises signaling the first signal S concurrently (optionally, only concurrently) with displaying the first sub-sequence of the sequence of frames of the video; and wherein indicating the first indicator I comprises indicating the first indicator I concurrently with displaying the successive second sub-sequence of the sequence of frames of the video.

In this example, signaling the first signal S concurrently with displaying the first sub-sequence of the sequence of frames of the video comprises signaling the first signal S only concurrently with displaying the first sub-sequence of the sequence of frames of the video.

In this example, indicating the first indicator I comprises indicating the first indicator I concurrently with displaying the first sub-sequence of the sequence of frames of the video and concurrently with displaying the successive second sub-sequence of the sequence of frames of the video.

In this example, detecting the first object O in the sequence of frames of the video comprises: detecting the first object O in a first frame of the sequence of frames of the video; and not detecting the first object O in a successive second frame of the sequence of frames of the video; wherein signaling the first signal S comprises signaling the first signal S concurrently (optionally, only concurrently) with displaying the first frame of the sequence of frames of the video; and wherein indicating the first indicator I comprises indicating the first indicator I concurrently with displaying the successive second frame of the sequence of frames of the video. In this way, the first indicator I indicates the first object O while or even though the detected object is not displayed.

In this example, signaling the first signal S concurrently with displaying the first frame of the sequence of frames of the video comprises signaling the first signal S only concurrently with displaying the first frame of the sequence of frames of the video.

In this example, indicating the first indicator I comprises indicating the first indicator I concurrently with displaying the first frame of the sequence of frames of the video and concurrently with displaying the successive second frame of the sequence of frames of the video.

In this example, indicating the first indicator I consecutively to displaying the detected first object O comprises displaying the first indicator I consecutively to displaying the detected first object O.

In this example, indicating the first indicator I consecutively to displaying the detected first object O comprises displaying the first indicator I consecutively to displaying the detected first object O, for example wherein the first indicator I comprises and/or is and/or is provided in the form of a visual indicator.

In this example, the method comprises compositing the sequence of frames of the video and the first indicator I, for example by overlaying the first indicator I and the sequence of frames of the video such as overlaying the first indicator I on the sequence of frames of the video, thereby providing a composite video.

In this example, displaying the first indicator I consecutively to displaying the detected first object O comprises overlaying the first indicator I and the sequence of frames of the video.

In this example, overlaying the first indicator I and the sequence of frames of the video comprises not occluding the detected first object O.

In this example, a frame of the sequence of frames comprises an image region IR, including the detected first object O, and a non-image region NIR, wherein overlaying the first indicator I and the sequence of frames of the video comprises overlaying the non-image region NIR.

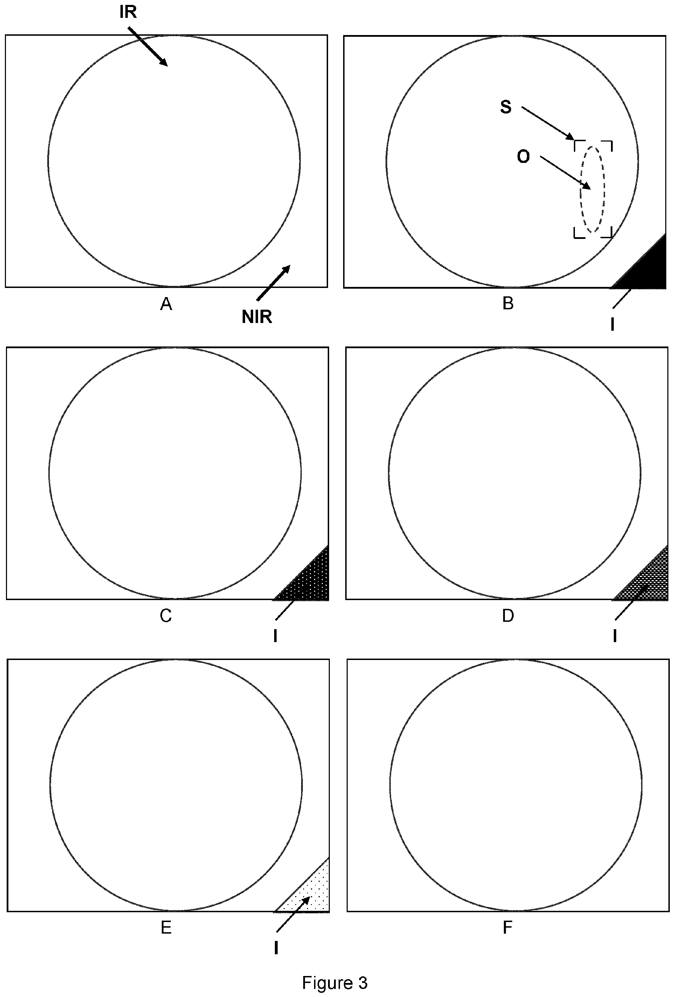

Figure 3 schematically depicts a method according to an exemplary embodiment. The method is generally as described with respect to Figure 2.

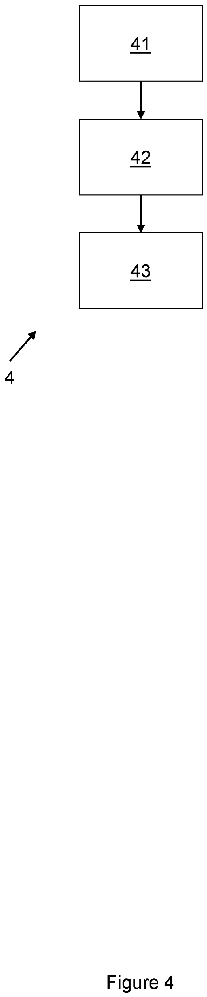

Figure 4 schematically depicts an apparatus 4 according to an exemplary embodiment.

The apparatus 4 comprises a camera 41, a display 42 and a computer 43, comprising a processor and a memory, configured to perform the method according to the first aspect, for example as described with respect to any of Figures 1 to 3.

Although a preferred embodiment has been shown and described, it will be appreciated by those skilled in the art that various changes and modifications might be made without departing from the scope of the invention, as defined in the appended claims and as described above.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A method implemented by a computer, comprising a processor and a memory, the method comprising:
receiving a sequence of frames of a video;
detecting a first object in the sequence of frames of the video; and
signaling a first signal, responsive to detecting the first object in the sequence of frames of the video;
**characterized by**:
displaying the sequence of frames of the video, comprising displaying the detected first object; and
indicating a first indicator, responsive to detecting the first object in the sequence of frames of the video;
wherein signaling the first signal comprises signaling the first signal concurrently with displaying the detected first object; and
wherein indicating the first indicator comprises indicating the first indicator consecutively to displaying the detected first object.

2. The method according to claim 1, wherein signaling the first signal comprises transiently signaling the first signal; and/or wherein indicating the first indicator comprises persistently indicating the first indicator.

3. The method according to any previous claim:
wherein detecting the first object in the sequence of frames of the video comprises: detecting the first object in a first sub-sequence of the sequence of frames of the video; and not detecting the first object in a successive second sub-sequence of the sequence of frames of the video;
wherein signaling the first signal comprises signaling the first signal concurrently with displaying the first sub-sequence of the sequence of frames of the video; and
wherein indicating the first indicator comprises indicating the first indicator concurrently with displaying the successive second sub-sequence of the sequence of frames of the video.

4. The method according to any previous claim, wherein indicating the first indicator comprises pointing from the current frame in the sequence of frames towards a preceding frame in the sequence of frames.

5. The method according to any previous claim, wherein indicating the first indicator comprises indicating the first indicator based on a separation between the current frame in the sequence of frames and a preceding frame, including the detected first object, in the sequence of frames.

6. The method according to claim 5, wherein indicating the first indicator based on the separation between the current frame in the sequence of frames and the preceding frame, including the detected first object, in the sequence of frames comprises and/or is changing an attribute of the first indicator based on the separation between the current frame in the sequence of frames and the preceding frame, including the detected first object, in the sequence of frames.

7. The method according to any of claims 5 to 6, wherein indicating the first indicator based on the separation between the current frame in the sequence of frames and the preceding frame, including the detected first object, in the sequence of frames comprises and/or is changing an attribute of the first indicator inversely proportionally to the separation between the current frame in the sequence of frames and the preceding frame, including the detected first object, in the sequence of frames.

8. The method according to any of claims 5 to 7, wherein the separation comprises and/or is a temporal separation and/or a spatial separation.

9. The method according to any previous claim, wherein signaling the first signal concurrently with displaying the detected first object comprises displaying the first signal concurrently with displaying the detected first object and/or wherein indicating the first indicator consecutively to displaying the detected first object comprises displaying the first indicator consecutively to displaying the detected first object.

10. The method according to claim 9, wherein displaying the first signal concurrently with displaying the detected first object comprises overlaying the first signal and the sequence of frames of the video, optionally wherein overlaying the first signal and the sequence of frames of the video comprises not occluding the detected first object.

11. The method according to any of claims 9 to 10, wherein displaying the first indicator consecutively to displaying the detected first object comprises overlaying the first indicator and the sequence of frames of the video, optionally wherein overlaying the first indicator and the sequence of frames of the video comprises not occluding the detected first object.

12. The method according to any previous claim, comprising:
detecting a second object in the sequence of frames of the video;
signaling a second signal, responsive to detecting the second object in the sequence of frames of the video; and
indicating a second indicator, responsive to detecting the second object in the sequence of frames of the video;
wherein displaying the sequence of frames of the video comprises displaying the detected second object;
wherein signaling the second signal comprises signaling the second signal concurrently with displaying the detected second object;
wherein indicating the second indicator comprises indicating the second indicator consecutively to displaying the detected second object; and
wherein indicating the first indicator comprises indicating the first indicator concurrently with displaying the detected second object.

13. The method according to any previous claim, wherein receiving the sequence of frames of the video comprises transitorily storing the sequence of frames of the video and/or wherein receiving the sequence of frames of the video excludes non-transitorily storing the sequence of frames of the video.

14. The method according to any previous claim, wherein the video comprises and/or is endoscopic video, for example gastrointestinal tract endoscopic video such as colonoscopy video, and wherein the first detected object comprises and/or is an endoscopic feature for example erythema, erosion, ulceration, edema, nodules, polyp and/or stenosis and/or an anatomical structure selected from a group consisting of healthy mucosa, stool, colonic fluid, blood vessel, inflamed mucosa, erosion, lesion and polyp.

15. A computer, comprising a processor and a memory, configured to perform the method according to any of claims 1 to 14; a computer program comprising instructions which, when executed by a computer, comprising a processor and a memory, cause the computer to perform the method according to any of claims 1 to 14; and/or a tangible computer-readable recording medium storing instructions which, when executed by a computer, comprising a processor and a memory, cause the computer to perform the method according to any of claims 1 to 14.
